(19) Europäisches Patentamt
European Patent Office
Office européen des brevets

(11) Veröffentlichungsnummer : **0 026 737**
**B1**

(12) **EUROPÄISCHE PATENTSCHRIFT**

(45) Veröffentlichungstag der Patentschrift : **09.03.83**

(51) Int. Cl.$^3$ : **C 07 D 213/61**, C 07 F 9/40

(21) Anmeldenummer : **80810276.8**

(22) Anmeldetag : **04.09.80**

(54) **Verfahren zur Herstellung von 2,3,5,6-Tetrachlorpyridin.**

(30) Priorität : **10.09.79 US 74217**

(43) Veröffentlichungstag der Anmeldung : **08.04.81 Patentblatt 81/14**

(45) Bekanntmachung des Hinweises auf die Patenterteilung : **09.03.83 Patentblatt 83/10**

(84) Benannte Vertragsstaaten : **CH DE FR GB LI**

(56) Entgegenhaltungen :
**US A 3 993 654**

**Chemical Abstracts Band 86, Nr. 25 20, Juni 1977 Columbus, Ohio, USA S. D. MOSHCHITSKII « 2,3,5,6-Tetrachloropyridine » Seite 591, Abstract Nr. 189723c.**

(73) Patentinhaber : **CIBA-GEIGY AG**
**Patentabteilung Postfach**
**CH-4002 Basel (CH)**

(72) Erfinder : **Weis, Claus D., Dr.**
**Bünenmattweg 3**
**CH-4148 Pfeffingen (CH)**
Erfinder : **Sutter, Peter**
**Seemättlistrasse 14/2**
**CH-4132 Muttenz (CH)**

**EP 0 026 737 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von 2,3,5,6-Tetrachlorpyridin

Die Erfindung betrifft ein Verfahren zur Herstellung von 2,3,5,6-Tetrachlorpyridin aus Pentachlorpyridin.

2,3,5,6-Tetrachlorpyridin ist ein wertvolles Handelsprodukt, das zur Herstellung von insektiziden Formulierungen verwendet werden kann. Ferner dient 2,3,5,6-Tetrachlorpyridin als Zwischenprodukt zur Herstellung von herbizid wirksamen α-[4-(3′, 5′, 6′-Trichlorpyrid-2′-yloxy)-phenoxy]-alkancarbonsäuren und deren Derivaten. Die Herstellung und Verwendung solcher α-[4-(3′, 5′, 6′-Trichlorpyrid-2′ -yloxy)-phenoxy]-alkancarbonsäuren und ihrer Derivate ist beispielsweise in der US-Patentschrift 4,133, 675 beschrieben.

Es ist bekannt, 2,3,5,6-Tetrachlorpyridin durch Umsetzung von Pentachlorpyridin mit Zink und Salzsäure in einem alkoholischen oder einem wässerigen Reaktionsmedium herzustellen. In wässerigem Reaktionsmedium wird die Umsetzung bei Temperaturen von 110 bis 160 °C unter dem der angewendeten Reaktionstemperatur entsprechenden Druck durchgeführt. Dabei werden Umsätze an Pentachlorpyridin von 82,5% bis 96,9% und Ausbeuten von 87,6% bis 94,6% erzielt (vgl. US-Patentschrift 3,993,654). Als Nebenprodukte treten Dichlorpyridine und Trichlorpyridine auf. Die Bildung dieser Nebenprodukte nimmt mit steigen dem Umsatz an Pentachlorpyridin zu.

Nach diesem bekannten Verfahren kann 2,3,5,6-Tetrachlorpyridin zwar in guter Ausbeute hergestellt werden, doch besitzt das Verfahren den Nachteil, dass es unter Druck durchgeführt werden muss und daher einen beträchtlichen apparativen Aufwand erfordert.

Der Erfindung liegt daher die Aufgabe zugrunde, ein Verfahren bereitzustellen, nach dem 2,3,5,6-Tetrachlorpyridin auf einfache Weise unter Normaldruck in guten Ausbeuten und hoher Reinheit hergestellt werden kann.

Gemäss Erfindung wird 2,3,5,6-Tetrachlorpyridin in der Weise hergestellt, dass man Pentachlorpyridin in einem Alkanphosphonsäuredialkylester (Dialkylalkanphosphonat) mit je 1 bis 4 Kohlenstoffatomen in den Alkylgruppen oder in einem Phosphorsäuretrialkylester (Trialkylphosphat) mit je 1 bis 4 Kohlenstoffatomen in den Alkylgruppen als Lösungsmittel bei 60 bis 120 °C in Gegenwart von 1,4 bis 2,8 Mol pro Mol Pentachlorpyridin eines Ammoniumsalzes einer anorganischen oder organischen Säure mit 1,2 bis 1,6 Grammatom Zink pro Mol Pentachlorpyridin umsetzt.

Geeignete Alkanphosphonsäuredialkylester, die erfindungsgemäss als Lösungsmittel verwendet werden können, sind beispielsweise die Dimethyl-, Diäthyl-, Di-n-Propyl-, Diisopropyl-, Di-n-Butyl-, Di-sec.-Butyl-, Diisobutyl- und Di-tert.-Butylester der Methan-, Äthan-, 1-Methyläthan-, 1,1-Dimethyläthan-, Propan-, 1-Methylpropan-, 2-Methylpropan- und Butanphosphonsäure. Bevorzugte Alkanphosphonsäuredialkylester sind Methanphosphonsäuredimethylester und Äthanphosphonsäurediäthylester.

Geeignete Phosphorsäuretrialkylester, die erfindungsgemäss als Lösungsmittel verwendet werden können, sind beispielsweise Phosphorsäuretrimethylester, Phosphorsäuretriäthylester, Phosphorsäuretri-n-propylester, Phosphorsäuretriisopropylester und Phosphorsäuretributylester. Bevorzugte Phosphorsäuretrialkylester sind Phosphorsäuretrimethylester und Phosphorsäuretriäthylester.

Innerhalb des Temperaturbereiches von 60 bis 120 °C, in dem das erfindungsgemässe Verfahren durchgeführt werden kann, sind Temperaturen von 85 bis 90 °C bevorzugt.

Die erfindungsgemäss verwendbaren Ammoniumsalze enthalten als Kation das Ammoniumion oder die davon durch teilweisen oder vollständigen Ersatz der Wasserstoffatome durch Alkyl- und/oder Phenylgruppen abgeleiteten Derivate, wobei die Phenylgruppen durch einfache Substituenten, wie Alkyl, Alkoxy oder Halogen, substituiert sein können. Als Anion enthalten die erfindungsgemäss verwendbaren Ammoniumsalze den Rest einer beliebigen anorganischen oder organischen Säure, die in der Lage ist, Ammoniumsalze zu bilden.

Vorteilhaft verwendbare Ammoniumsalze entsprechen der alljemeinen Formel I

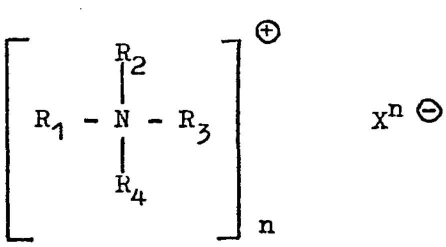

(I)

in welcher $R_1$, $R_2$, $R_3$ und $R_4$ gleich oder verschieden sein können, und je Wasserstoff, Alkyl mit 1 bis 4 Kohlenstoffatomen oder Phenyl, das gegebenenfalls durch Halogen, Alkylgruppen mit 1 bis 4 Kohlenstoffatomen oder Alkoxygruppen mit 1 bis 4 Kohlenstoffatomen substituiert sein kann, bedeuten, X für ein Anion aus der Gruppe Chlorid, Bromid, Sulfat, Hydrogensulfat, Phosphat, Hydrogenphosphat, Dihydrogenphosphat, Carbonat, Hydrogencarbonat, Acetat, Propionat, Butyrat, Isobutyrat, Oxalat, Benzoat, Alkanphosphonat mit 1 bis 4 Kohlenstoffatomen in der Alkylgruppe und Alkan- bzw. Benzolsulphonat mit

1 bis 4 Kohlenstoffatomen in der Alkylgruppe steht und n 1 bis 3 bedeutet und der Zahl der negativen Ladungen des jeweiligen Anions X entspricht.

Als weitere vorteilhaft zu verwendende Ammoniumsalze sind die Ammoniumsalze des Methanphosphonsäuremonomethylesters der allgemeinen Formel II

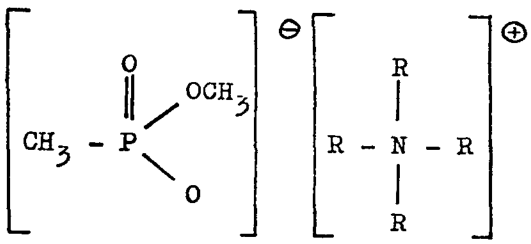

(II)

in welcher R Wasserstoff oder Methyl bedeutet, zu nennen. Diese Ammoniumsalze stellen neue Verbindungen dar, die in einfacher Weise durch Erhitzen von Ammoniumchlorid oder Tetramethylammoniumchlorid in Methanphosphonsäuredimethylester auf 150 °C unter Abspaltung von Methylchlorid erhalten werden können. Die Umsetzung wird vorteilhaft in überschüssigem Methanphosphonsäuredimethylester als Lösungsmittel durchgeführt. Nach beendigter Umsetzung wird der überschüssige Methanphosphonsäuredimethylester im Vakuum abdestilliert und das Ammoniumsalz, gegebenenfalls nach Digerieren in einem geeigneten Lösungsmittel, z. B. Aceton, Methyläthylketon oder Aether, in kristalliner Form gewonnen.

Bevorzugte Ammoniumsalze sind Ammoniumchlorid, Ammoniumsulfat, Ammoniumcarbonat, das Ammoniumsalz des Methanphosphonsäuremonomethylesters, sowie das Tetramethylammoniumsalz des Methanphosphonsäuremonomethylesters.

Die vorgenannten Ammoniumsalze werden bei Verwendung von Methanphosphonsäuredialkylester als Lösungsmittel vorzugsweise in einer Menge von 1,6 Mol pro Mol Pentachlorpyridin eingesetzt. Bei Verwendung von Äthanphosphonsäuredialkylester oder Trialkylphosphat als Lösungsmittel werden vorzugsweise 2,6 bis 2,8 Mol Ammoniumsalz pro Mol Pentachlorpyridin eingesetzt.

Das erfindungsgemäss zu verwendende Zink wird in Form von Zinkspänen oder vorzugsweise in Form von Zinkstaub eingesetzt. Es werden vorzugsweise 1,2-1,3 Grammatome Zink pro Mol Pentachlorpyridin verwendet.

Durch das erfindungsgemässe Verfahren wird es möglich, die selektive Dechlorierung von Pentachlorpyridin zu 2,3,5,6-Tetrachlorpyridin bei Normaldruck durchzuführen. Dabei wird das 2,3,5,6-Tetrachlorpyridin in einer Ausbeute von etwa 92% d. Th. und in einer Reinheit von 97% erhalten.

Das erfindungsgemässe Verfahren wird durch die folgenden Beispiele näher erläutert.

## Beispiel 1

12,76 g (0,05 Mol) Pentachlorpyridin werden unter Erwärmen auf 90 °C in 80 ml Dimethylmethanphosphonat gelöst. In die klare Lösung wird nach Zugabe von 4,1 g (0,063 Grammatom) Zinkstaub unter starkem Rühren eine Lösung von 4,26 g (0,08 Mol) Ammoniumchlorid in 15 ml Wasser während 20 Minuten eingetropft. Nach beendigter Zugabe der Ammoniumchlorid-Lösung wird 40 Minuten nachgerührt. Dann wird das erhaltene Reaktionsgemisch heiss filtriert und der Filterrückstand mit 10 ml Dimethylmethanphosphonat gewaschen. Das Filtrat wird auf 500 ml Eiswasser gegossen, mit 12,5 ml konz. Salzsäure versetzt und 2 Stunden gerührt. Danach wird das in Form von weissen Kristallen abgeschiedene 2,3,5,6-Tetrachlorpyridin abfiltriert, mit 150 ml Wasser gewaschen und getrocknet. Auf diese Weise werden 10,0 g (90,8% d. Th.) 2,3,5,6-Tetrachlorpyridin vom Schmelzpunkt 89 bis 90 °C erhalten. Das Produkt enthält nach gaschromatographischer Analyse 97,0% 2,3,5,6-Tetrachlorpyridin, 0,5% 2,3,5-Trichlorpyridin, 0,6% 2,3,6-Trichlorpyridin und 1,6% Pentachlorpyridin.

## Beispiel 2

In eine auf 80 °C erwärmte Lösung von 5,1 g (0,02 Mol) Pentachlorpyridin in 35 ml Dimethylmethanphosphonat werden 1,84 g (0,028 1 Grammatom) Zinkstaub eingetragen. Anschliessend wird bei einer Temperatur von 90 bis 95 °C unter starkem Rühren eine Lösung von 2,44 g (0,025 4 Mol) Ammoniumcarbonat in 10 ml Wasser während 70 Minuten eingetropft. Dann wird das Reaktionsgemisch in 250 ml Eiswasser eingerührt, mit 5 ml konz. Salzsäure versetzt und 10 Minuten gerührt. Anschliessend wird die Mischung zweimal mit je 100 ml Aether extrahiert. Die vereinigten Extrakte werden mit 70 ml Wasser gewaschen, über Natriumsulfat getrocknet, filtriert und zur Trockene eingedampft. Als Rückstand werden 4,05 g (92% d. Th.) 2,3,5,6-Tetrachlorpyridin vom Schmelzpunkt 86 bis 88 °C erhalten. Das Produkt enthält nach gaschromatographischer Analyse 93,4% 2,3,5,6-Tetrachlorpyridin, 2,1% 2,3,5-Trichlorpyridin, 2,2% 2, 3,6-Trichlorpyridin und 0,8% Pentachlorpyridin.

Beispiel 3

Bei der Umsetzung von 12,76 g (0,05 Mol) Pentachlorpyridin mit 4,1 g (0,063 Grammatom) Zinkstaub und 11,6 g (0,08 Mol) Ammoniumsulfat nach der in Beispiel 1 beschriebenen Methode werden 10,1 g (92% d. Th.) rohes 2,3,5,6-Tetrachlorpyridin erhalten, das nach gaschromatographischer Analyse 92% 2,3,5,6-Tetrachlorpyridin, 3,3% 2,3,5-Trichlorpyridin, 1,3% 2,3,6-Trichlorpyridin und 2,9% Pentachlorpyridin enthält.

Beispiel 4

Bei der Umsetzung von 12,76 g (0,05 Mol) Pentachlorpyridin mit 4,1 g (0,063 Grammatom) Zinkstaub und 10,6 g (0,08 Mol) Diammoniumhydrogenphosphat nach der in Beispiel 1 beschriebenen Methode werden 10,2 g (93% d. Th.) rohes 2,3,5,6-Tetrachlorpyridin erhalten, das nach gaschromatographischer Analyse 95,1% 2,3,5,6-Tetrachlorpyridin, 0,8% 2,3,5-Trichlorpyridin, 0,5% 2,3,6-Trichlorpyridin und 3,6% Pentachlorpyridin enthält.

Beispiel 5

In eine auf 90 °C erwärmte Lösung von 12,57 g (0,05 Mol) Pentachlorpyridin in 150 ml Dimethylmethanphosphonat wird nach Zugabe von 4,6 g (0,07 Grammatom) Zinkstaub unter Rühren eine Lösung von 9,52 g (0,075 Mol) Ammoniumsalz des Methanphosphonsäuremonomethylesters in 44 ml Wasser während 25 Minuten eingetropft. Nach beendigter Zugabe des Ammoniumsalzes wird das Reaktionsgemisch zunächst 30 Minuten nachgerührt und anschliessend heiss filtriert und der Filterrückstand mit 10 ml Dimethylmethanphosphonat gewaschen. Das Filtrat wird in 500 ml Eiswasser gegossen, mit 12,5 ml konz. Salzsäure versetzt und 2 Stunden gerührt. Danach werden die abgeschiedenen Kristalle abfiltriert, mit 150 ml Wasser gewaschen und getrocknet. Es werden 8,72 g (80,4% d. Th.) rohes 2,3,5,6-Tetrachlorpyridin vom Schmelzpunkt 88 bis 89 °C erhalten, das nach gaschromaoographischer Analyse 94,1% 2,3,5,6-Tetrachlorpyridin, 2,5% 2,3,5-Trichlorpyridin, 1,9% 2,3,6-Trichlorpyridin und 0,17% Pentachlorpyridin enthält.

Das Ammoniumsalz des Methanphosphonsäuremonomethylesters kann wie folgt hergestellt werden :

In einem 1 Liter Rundkolben mit aufgesetztem Rückflusskühler werden 540 g (4 Mol) Dimethylmethanphosphonat und 107 g (2 Mol) Ammoniumchlorid unter Rühren auf 110 °C erhitzt, wobei die Reaktion unter Entwicklung von Methylchlorid einsetzt. Die Temperatur wird dann innert 20 Minuten auf 138 °C und anschliessend weitere 20 Minuten auf 151 °C erhöht. Danach wird die klare, farblose Lösung bei 12 Torr. zur Trockene eingedampft. Als Rückstand wird ein gelbes Oel erhalten, welches nach Zugabe von 500 ml Aceton 24 Stunden gerührt wird, wobei Kristallisation eintritt. Die Kristallsuspension wird auf 0 °C gekühlt, filtriert und anschliessend auf dem Filter unter Ausschluss von Feuchtigkeit mit 150 ml Aether gewaschen. Nach dem Trocknen bei 50 °C und 12 Torr. erhält man 118,6 g (46,7% d. Th.) des Ammoniumsalzes des Methanphosphonsäuremonoethylesters vom Schmelzpunkt 96 bis 103 °C als stark hygroskopische Kristalle, die beim Stehen an der Luft bald zerfliessen.

Beispiel 6

Eine Suspension von 12,8 g (0,05 Mol) Pentachlorpyridin und 4,1 g (0,062 Grammatom) Zinkstaub in 120 ml Dimethylmethanphosphonat wird unter Rühren auf 80 °C erwärmt. Dann wird innerhalb 15 Minuten eine Lösung von 13,76 g (0,075 Mol) des Tetramethylammoniumsalzes des Methanphosphonsäuremonomethylesters in 30 ml Wasser eingetropft. Anschliessend wird heiss filtriert, der Filterrückstand mit 30 ml Dimethylmethanphosphonat gewaschen, und das Filtrat in 600 ml Eiswasser gegossen, das 12,5 ml konz. Salzsäure enthält. Die hierbei entstehende weisse Kristallsuspension wird 30 Minuten gerührt. Anschliessend wird filtriert, der Filterrückstand mit Wasser gewaschen und getrocknet. Es werden 9,95 g (90% d. Th.) rohes 2,3,5,6-Tetrachlorpyridin vom Schmelzpunkt 87,5 bis 89 °C erhalten, das nach gaschromatographischer Analyse 96,9% 2,3,5,6-Tetrachlorpyridin, 1,6% 2,3,5-Trichlorpyridin, 0,9% 2,3,6-Trichlorpyridin und 0,6% Pentachlorpyridin enthält.

Das verwendete Tetramethylammoniumsalz des Methanphosphonsäuremonomethylesters kann wie folgt hergestellt werden :

In einem 1 Liter Kolben mit aufgesetztem Rückflusskühler werden 540 g (4 Mol) Dimethylmethanphosphonat und 219 g (2 Mol) Tetramethylammoniumchlorid unter Rühren auf 130 °C erhitzt, wobei die Reaktion unter Entwicklung von Methylchlorid einsetzt. Die Temperatur wird nun innerhalb 3 Stunden auf 150 °C gesteigert. Danach wird das Reaktionsgemisch noch eine weitere Stunde auf 160 °C erhitzt. Die klare farblose Lösung wird bei 12 Torr. zur Trockne eingedampft und der weisse kristalline Rückstand wird mit 400 ml Aceton versetzt, 30 Minuten bei 0 °C gerührt und filtriert. Das Filtergut wird mit 500 ml Aether gewaschen und bei 60 °C und 12 Torr über festem Kaliumhydroxid getrocknet. Es werden 312 g (85% d. Th.) Tetramethylammoniumsalz des Methanphosphonsäuremonomethylesters vom Schmelzpunkt 172 bis 177 °C (Zers.) in Form von weissen Kristallen erhalten.

Beispiel 7

Eine Suspension von 8,22 g (0,075 Mol) Tetramethylammoniumchlorid in 90 ml Dimethylmethanphosphonat wird 1 Stunde auf 160 °C erhitzt. Dann wird auf 90 °C abgekühlt und 12,57 g (0,05 Mol) Pentachlorpyridin und 3 ml Wasser zugegeben. Dann werden während 30 Minuten 4,1 g (0,063 Grammatom) Zinkstaub portionsweise eingetragen. Nach beendigter Zugabe des Zinkstaubes wird zunächst 20 Minuten nachgerührt und dann filtriert. Das Filtrat wird in eine Lösung von 12,5 ml konz. Salzsäure in 500 ml Wasser einfliessen lassen und 2 Stunden gerührt. Durch Filtrieren, Waschen des Filtergutes mit Wasser und Trocknen werden 9,1 g (83,9% d. Th.) rohes 2,3,5,6-Tetrachlorpyridin vom Schmelzpunkt 88 bis 89 °C erhalten, das nach gaschromatographischer Analyse 91,25% 2,3,5,6-Tetrachlorpyridin, 2,66% 2,3,5-Trichlorpyridin, 2,59% 2,3,6-Trichlorpyridin und 0,5% Pentachlorpyridin enthält.

Beispiel 8

Nach der in Beispiel 1 beschriebenen Methode werden 12,76 g (0,05 Mol) Pentachlorpyridin mit 4,1 g (0,063 Grammatom) Zinkstaub und 7,22 g (0,135 Mol) Ammoniumchlorid umgesetzt, wobei jedoch anstelle von Dimethylmethanphosphonat Diäthyläthanphosphonat als Lösungsmittel verwendet wird. Es werden 10,2 g (93% d. Th.) rohes 2,3,5,6-Tetrachlorpyridin vom Schmelzpunkt 86 bis 88 °C erhalten, das nach gaschromatographischer Analyse 92,7% 2,3,5,6-Tetrachlorpyridin, 1,2% 2,3,5-Trichlorpyridin, 2,2% 2,3,6-Trichlorpyridin und 2,8% Pentachlorpyridin enthält.

Beispiel 9

Nach der in Beispiel 2 beschriebenen Methode werden 12,76 g (0,05 Mol) Pentachlorpyridin mit 4,1 g (0,063 Grammatom) Zinkstaub und 13,21 g (0,138 Mol) Ammoniumcarbonat umgesetzt, wobei jedoch Diäthyläthanphosphonat anstelle von Dimethylmethanphosphonat als Lösungsmittel verwendet wird. Es werden 10,3 g (93,5% d. Th.) 2,3,5,6-Tetrachlorpyridin vom Schmelzpunkt 86 bis 88 °C erhalten.

Beispiel 10

In eine auf 80 °C erwärmte Lösung von 5,1 g (0,02 Mol) Pentachlorpyridin in 35 ml Trimethylphosphat werden 1,7 g (0,027 Grammatom) Zinkstaub eingetragen und anschliessend unter starkem Rühren eine Lösung von 2,84 g (0,055 Mol) Ammoniumchlorid in 10 ml Wasser während 1 Stunde bei 80 bis 83 °C eingetropft. Anschliessend lässt man das Reaktionsgemisch in 250 ml Eiswasser einfliessen, fügt 5 ml konz. Salzsäure hinzu und rührt 10 Minuten nach. Dann wird die erhaltene Mischung zweimal mit je 100 ml Aether extrahiert, die vereinigten Aetherextrakte mit 70 ml Wasser gewaschen und über Natriumsulfat getrocknet. Durch Abdampfen des Aethers werden 4,1 g (93,2% d. Th.) rohes 2,3,5,6-Tetrachlorpyridin vom Schmelzpunkt 86 bis 88 °C erhalten, das nach gaschromatographischer Analyse 94,1% 2,3,5,6-Tetrachlorpyridin, 2,0% 2,3,5-Trichlorpyridin, 2,0% 2,3,6-Trichlorpyridin und 2,8% Pentachlorpyridin enthält.

Beispiel 11

Nach der in Beispiel 10 beschriebenen Methode werden 5,1 g (0,02 Mol) Pentachlorpyridin mit 1,7 g (0,027 Grammatom) Zinkstaub und 5,3 g (0,055 Mol) Ammoniumcarbonat umgesetzt. Es werden 4,1 g (93,2% d. Th.) 2,3,5,6-Tetrachlorpyridin vom Schmelzpunkt 86 bis 88 °C erhalten.

Beispiel 12

Nach der in Beispiel 10 beschriebenen Methode werden 5,1 g (0,02 Mol) Pentachlorpyridin mit 1,7 g (0,027 Grammatom) Zinkstaub und 2,84 g (0,055 Mol) Ammoniumchlorid umgesetzt, wobei jedoch Triäthylphosphat anstelle von Trimethylphosphat als Lösungsmittel verwendet wird. Es werden 4,05 g (93,1% d.Th.) 2,3,5,6-Tetrachlorpyridin vom Schmelzpunkt 86 bis 88 °C erhalten.

Beispiel 13

Nach der in Beispiel 11 beschriebenen Methode werden 5,1 g (0,002 Mol) Pentachlorpyridin mit 1, 7 g (0,027 Grammatom) Zinkstaub und 5,3 g (0,055 Mol) Ammoniumcarbonat umgesetzt, wobei jedoch Triäthylphosphat anstelle von Trimethylphosphat als Lösungsmittel verwendet wird. Es werden 4,1 g (93, 2% d. Th.) 2,3,5,6-Tetrachlorpyridin vom Schmelzpunkt 86 bis 88 °C erhalten.

**Ansprüche**

1. Verfahren zur Herstellung von 2,3,5,6-Tetrachlorpyridin durch Dechlorierung von Pentachlorpyridin, dadurch gekennzeichnet, dass man Pentachlorpyridin in einem Alkanphosphonsäuredialkylester mit je 1 bis 4 Kohlenstoffatomen in den Alkylgruppen oder in einem Phosphorsäuretrialkylester mit je 1 bis 4 Kohlenstoffatomen in den Alkylgruppen als Lösungsmittel bei 60 bis 120 °C in Gegenwart von 1,4 bis 2, 8 Mol pro Mol Pentachlorpyridin eines Ammoniumsalzes einer anorganischen oder organischen Säure mit 1,2 bis 1,6 Grammaton Zink pro Mol Pentachlorpyridin imte Normaldruck umsetzt.

2. Verfahren nach Anspruch 1, dadurch gekennzeichnet, dass man als Alkanphosphonsäuredialkylester Methanphosphonsäuredimethylester oder Äthanphosphonsäurediäthylester verwendet.

3. Verfahren nach Anspruch 1, dadurch gekennzeichnet, dass man als Phosphorsäuretrialkylester Phosphorsäuretrimethylester oder Phosphorsäuretriäthylester verwendet.

4. Verfahren nach Anspruch 1, dadurch gekennzeichnet, dass man die Dechlorierung des Pentachlorpyridins bei 85 bis 90 °C durchführt.

5. Verfahren nach Anspruch 1, dadurch gekennzeichnet, dass man ein Ammoniumsalz der allgemeinen Formel I

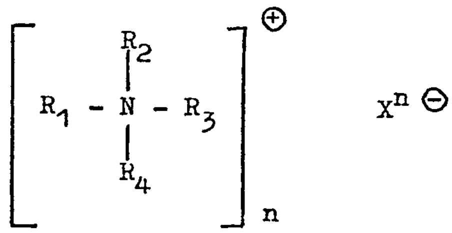

(I)

in welcher $R_1$, $R_2$, $R_3$ und $R_4$ gleich oder verschieden sein können, und je Wasserstoff, Alkyl mit 1 bis 4 Kohlenstoffatomen oder Phenyl, das gegebenenfalls durch Halogen, Alkylgruppen mit 1 bis 4 Kohlenstoffatomen oder Alkoxygruppen mit 1 bis 4 Kohlenstoffatomen substituiert sein kann, bedeuten, X für ein Anion aus der Gruppe Chlorid, Bromid, Sulfat, Hydrogensulfat, Phosphat, Hydrogenphosphat, Dihydrogenphosphat, Carbonat, Hydrogencarbonat, Acetat, Propionat, Butyrat, Isobutyrat, Oxalat, Benzoat, Alkanphosphonat mit 1 bis 4 Kohlenstoffatomen in der Alkylgruppe und Alkan- bzw. Benzolsulfonat mit 1 bis 4 Kohlenstoffatomen in der Alkylgruppe steht und n 1 bis 3 bedeutet und der Zahl der negativen Ladungen des jeweiligen Anions X entspricht.

6. Verfahren nach Anspruch 1, dadurch gekennzeichnet, dass man ein Ammoniumsalz der O-Methylmethanphosphonsäure der allgemeinen Formel II

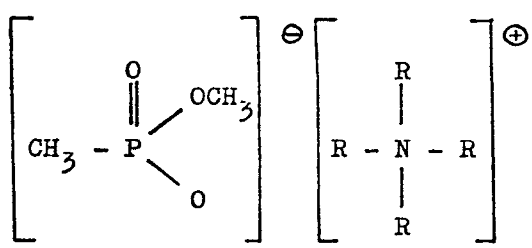

(II)

in welcher R Wasserstoff oder Methyl bedeutet, verwendet.

7. Verfahren nach Anspruch 1, dadurch gekennzeichnet, dass man als Ammoniumsalz Ammoniumchlorid, Ammoniumsulfat, Ammoniumcarbonat, das Ammoniumsalz des Methanphosphonsäuremonomethylesters oder das Tetramethylammoniumsalz des Methanphosphonsäuremonomethylesters verwendet.

8. Verfahren nach Anspruch 1, dadurch gekennzeichnet, dass man bei Verwendung von Methanphosphonsäuredialkylester als Lösungsmittel 1,6 Mol Ammoniumsalz pro Mol Pentachlorpyridin einsetzt.

9. Verfahren nach Anspruch 1, dadurch gekennzeichnet, dass man bei Verwendung von Äthanphosphonsäuredialkylester oder Trialkylphosphat als Lösungsmittel 2,6 bis 2,8 Mol Ammoniumsalz pro Mol Pentachlorpyridin einsetzt.

10. Verfahren nach Anspruch 1, dadurch gekennzeichnet, dass man 1,2-1,3 Grammatom Zink pro Mol Pentachlorpyridin einsetzt.

**Claims**

1. A process for producing 2,3,5,6-tetrachloropyridine by dechlorination of pentachloropyridine, which process comprises reacting pentachloropyridine, in an alkanephosphonic acid dialkyl ester having 1 to 4 carbon atoms in each of the alkyl groups or in a phosphoric acid trialkyl ester having 1 to 4 carbon

atoms in each of the alkyl groups as the solvent, at 60° to 120 °C under normal pressure, in the presence of 1.4 to 2.8 mols, per mol of pentachloropyridine, of an ammonium salt of an inorganic or organic acid, with 1.2 to 1.6 gram atoms of zinc per mol of pentachloropyridine.

2. A process according to Claim 1, wherein the alkanephosphonic acid dialkyl ester used is methanephosphonic acid dimethyl ester or ethanephosphonic acid diethyl ester.

3. A process according to Claim 1, wherein the phosphoric acid trialkyl ester used is phosphoric acid trimethyl ester or phosphoric acid triethyl ester.

4. A process according to Claim 1, wherein the dechlorination of the pentachloropyridine is performed at 85 to 90 °C.

5. A process according to Claim 1, wherein there is used an ammonium salt of the general formula I

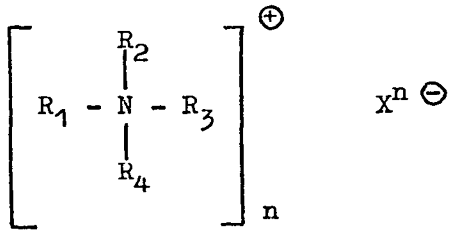

(I)

in which $R_1$, $R_2$, $R_3$ and $R_4$ can be identical or different and are each hydrogen, alkyl having 1 to 4 carbon atoms, or phenyl which can be substituted by halogen, by alkyl groups having 1 to 4 carbon atoms or by alkoxy groups having 1 to 4 carbon atoms, X is an anion from the group : chloride, bromide, sulfate, hydrogen sulfate, phosphate, hydrogen phosphate, dihydrogen phosphate, carbonate, hydrogen carbonate, acetate, propionate, butyrate, isobutyrate, oxalate, benzoate, alkanephosphonate having 1 to 4 carbon atoms in the alkyl group and alkane- or benzene sulfonate having 1 to 4 carbon atoms in the alkyl group, and n is 1 to 3 and corresponds to the number of negative charges of the respective anion X.

6. A process according to Claim 1, wherein there is used an ammonium salt of O-methyl-methanephosphonic acid of the general formula II

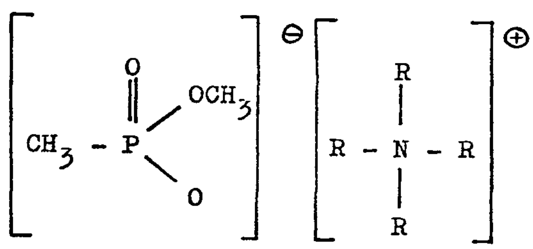

(II)

in which R is hydrogen or methyl.

7. A process according to Claim 1, wherein the ammonium salt used is : ammonium chloride, ammonium sulfate, ammonium carbonate, the ammonium salt of methanephosphonic acid monomethyl ester or the tetramethyl-ammonium salt of methanephosphonic acid monomethyl ester.

8. A process according to Claim 1, wherein, with the use of methanephosphonic acid dialkyl ester as solvent, there are used 1.6 mols of ammonium salt per mol of pentachloropyridine.

9. A process according to Claim 1, wherein, with the use of ethanephosphonic acid dialkyl ester or trialkyl phosphate as solvent, there are used 2.6 to 2.8 mols of ammonium salt per mol of pentachloropyridine.

10. A process according to Claim 1, wherein 1.2 to 1.3 gram atoms of zinc are used per mol of pentachloropyridine.

**Revendications**

1. Procédé de préparation de la 2,3,5,6-tétrachloropyridine par déchloration de la pentachloropyridine, caractérisé en ce que l'on fait réagir la pentachloropyridine dans un alcane-phosphonate de dialkyle contenant 1 à 4 atomes de carbone dans chacun des groupes alkyles ou dans un phosphate de trialkyle contenant 1 à 4 atomes de carbone dans chacun des groupes alkyles, qui sert de solvant, à une température de 60 à 120 °C, en présence de 1,4 à 2,8 moles, par mole de pentachloropyridine, d'un sel d'ammonium d'acide organique ou minéral, avec 1,2 à 1,6 atome-gramme de zinc par mole de pentachloropyridine, à pression normale.

2. Procédé selon la revendication 1, caractérisé en ce que l'on utilise en tant qu'alcane-phosphonate

de dialkyle le méthane-phosphonate de diméthyle ou l'éthane-phosphonate de diéthyle.

3. Procédé selon la revendication 1, caractérisé en ce que l'on utilise en tant que phosphate de trialkyle le phosphate de triméthyle ou le phosphate de triéthyle.

4. Procédé selon la revendication 1, caractérisé en ce que la déchloration de la pentachloropyridine est effectuée à une température de 85 à 90 °C.

5. Procédé selon la revendication 1, caractérisé en ce que l'on utilise un sel d'ammonium de formule générale I

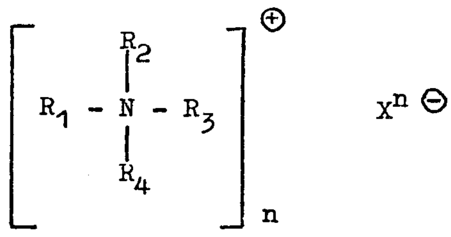 (I)

dans laquelle $R_1$, $R_2$, $R_3$ et $R_4$, qui peuvent avoir des significations identiques ou différentes, représentent chacun l'hydrogène, un groupe alkyle en C1-C4 ou phényle, lui-même éventuellement substitué par des halogènes, des groupes alkyles en C1-C4 ou alcoxy en C1-C4, X représente un anion pris dans le groupe formé par les anions chlorure, bromure, sulfate, bisulfate, phosphate, hydrogénophosphate, dihydrogénophosphate, carbonate, bicarbonate, acétate, propionate, butyrate, isobutyrate, oxalate, benzoate, alcane-phosphonate contenant 1 à 4 atomes de carbone dans le groupe alkyle et alcane- ou benzène-sulfonate contenant 1 à 4 atomes de carbone dans le groupe alkyle, et n a une valeur de 1 à 3 et correspond au nombre des charges négatives de l'anion X particulier.

6. Procédé selon la revendication 1, caractérisé en ce que l'on utilise un sel d'ammonium de l'acide O-méthyl-méthane-phosphonique de formule générale II

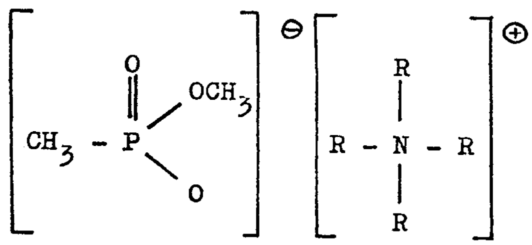 (II)

dans laquelle R représente l'hydrogène ou le groupe méthyle.

7. Procédé selon la revendication 1, caractérisé en ce que l'on utilise en tant que sel d'ammonium le chlorure d'ammonium, le sulfate d'ammonium, le carbonate d'ammonium, le sel d'ammonium du méthane-phosphonate de monométhyle ou le sel de tétraméthylammonium du méthane-phosphonate de monométhyle.

8. Procédé selon la revendication 1, caractérisé en ce que, lorsque le solvant est un méthane-phosphonate de dialkyle, on utilise 1,6 moles de sel d'ammonium par mole de pentachloropyridine.

9. Procédé selon la revendication 1, caractérisé en ce que, lorsque le solvant est un éthane-phosphonate de dialkyle ou un phosphate de trialkyle, on utilise de 2,6 à 2,8 moles de sel d'ammonium par mole de pentachloropyridine.

10. Procédé selon la revendication 1, caractérisé en ce que l'on utilise de 1,2 à 1,3 atome-grammes de zinc par mole de pentachloropyridine.